(19) Europäisches Patentamt
European Patent Office
Office européen des brevets

(11) Veröffentlichungsnummer: **0 034 256**
**A2**

(12) **EUROPÄISCHE PATENTANMELDUNG**

(21) Anmeldenummer: **81100327.6**

(22) Anmeldetag: **17.01.81**

(51) Int. Cl.³: **C 07 C 143/38**
C 07 C 143/52
//C07D263/56

(30) Priorität: **31.01.80 DE 3003539**

(43) Veröffentlichungstag der Anmeldung: **26.08.81 Patentblatt 81/34**

(84) Benannte Vertragsstaaten: **CH DE FR GB IT LI**

(71) Anmelder: **BAYER AG**
**Zentralbereich Patente, Marken und Lizenzen**
**D-5090 Leverkusen 1, Bayerwerk(DE)**

(72) Erfinder: **Bremen, Josef, Dr.**
**Am Kettnersbusch 1 e**
**D-5090 Leverkusen 3(DE)**

(72) Erfinder: **Dorlars, Alfons, Dr.**
**Mozartstrasse 32**
**D-5090 Leverkusen 1(DE)**

(54) **4-Phenylbenzaldehyd-2-sulfonsäuren sowie deren Herstellung.**

(57) Verbindungen der Formel

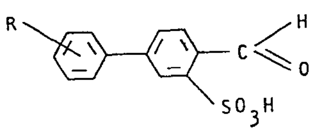

worin
R Wasserstoff, Halogen, eine gegebenenfalls abgewandelte Carbonsäure- oder Sulfonsäuregruppe ist, erhält man beispielsweise dadurch, daß man in Verbindungen der Formel

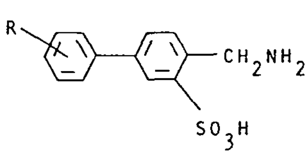

die Aminomethylgruppe nach Sommelet in die Aldehydgruppe umwandelt.

Die neuen Aldehyde sind Ausgangsstoffe zur Herstellung wertvoller optischer Aufheller vom Stilben-Typ.

EP 0 034 256 A2

0034256

BAYER AKTIENGESELLSCHAFT 5090 Leverkusen, Bayerwerk
Zentralbereich
Patente, Marken und Lizenzen K/Kü-c

# 4-Phenylbenzaldehyd-2-sulfonsäuren sowie deren Herstellung

Gegenstand der Erfindung sind Benzaldehydderivate, die in Form der freien Säure der Formel

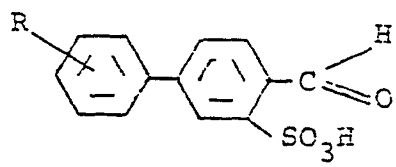

(I)

entsprechen, worin R Wasserstoff, Halogen, eine gegebenenfalls abgewandelte Carbonsäure- oder Sulfonsäuregruppe ist.

Vorzugsweise steht R für Wasserstoff.

Geeignete Reste von Carbonsäure- bzw. Sulfonsäurederivaten sind solche der Formeln COOV, $CON(W)_2$, $SO_2OV$ und $SO_2N(W)_2$ , wobei V für $C_1$-$C_4$-Alkyl, Aralkyl oder Aryl und W für V oder H stehen.

Geeignete Halogenatome sind F, Br und vor allem Cl.

Geeignete Arylreste sind Phenyl, Tolyl, Chlorphenyl u.a.

Die Verbindungen der Formel I können auf verschiedene Weise hergestellt werden.

Ein Verfahren ist dadurch gekennzeichnet, daß man in Verbindungen der Formel (II)

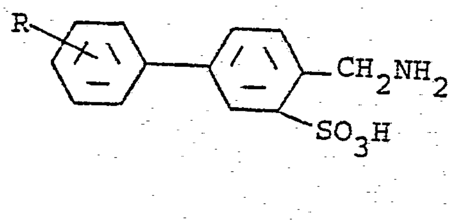

(II)

in der R die oben angegebene Bedeutung hat, die Aminomethylgruppe in an sich bekannter Weise durch Umsetzung mit Hexamethylentetramin oder Hexamethylentetramin Formaldehyd- Gemischen in die Aldehydgruppe umwandelt (vgl. Angyal, The Sommelet Reaction, in Organic Reactions, Vol. 8, p 197 ff).

Anstelle der Aminomethyl- Verbindungen der Formel II können grundsätzlich auch die entsprechenden Chlormethylverbindungen eingesetzt werden, die jedoch im allgemeinen schwer zugänglich sind (siehe weiter unten).

Zur Oxidation der Aminomethylverbindung nach "Sommelet" verwendet man mindestens äquivalente Mengen, vorzugsweise einen Überschuß Urotropin oder Urotropin-Formalin-Gemisch.

Als Reaktionsmedium können verdünnte Alkohole oder verdünnte Essigsäure verwendet werden, wobei als Alkohole Methanol, Ethanol, Propanol, Isopropanol oder Glykolmonomethyläther in Frage kommen und die Konzentrationen der verdünnten Alkohole bei 40 bis 70 %, die der verdünnten Essigsäure bei 50 bis 80 % liegen sollten.

Bei der Durchführung der Oxidation in verdünntem Alkohol sollte durch Zugabe von verdünnter Mineralsäure ein pH-Wert von 3 - 5 zu Beginn der Umsetzung eingestellt werden.

Als Reaktionstemperatur wählt man vorteilhaft die Rückflußtemperatur des verwendeten Reaktionsmediums.

Die Oxidation wird im allgemeinen so durchgeführt, daß zu einer Suspension der Aminomethylverbindung der Formel (II) in 80 %iger Essigsäure bei Raumtemperatur zunächst ein Überschuß von ca. 50 Mol % Formaldehyd in Form einer wäßrigen 30 %igen Formalinlösung und dann Urotropin im Überschuß von 10 - 40 Mol % gegeben wird. Dann wird 4 bis 8 Stunden zum Rückfluß erhitzt.

Ein weiteres Verfahren zur Herstellung der neuen Benzaldehyde ist dadurch gekennzeichnet, daß man Hydroxymethylverbindungen der Formel

BAD ORIGINAL

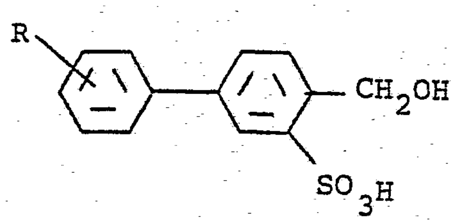

(III)

in ebenfalls an sich bekannter Weise zum entsprechenden Aldehyd oxidiert, (vgl. Houben-Weyl, Bd. VII/1, Seite 135 ff).

Geeignete Oxidationsmittel sind z.B. Chromsäure, Bichromat/Schwefelsäure, Salpetersäure, Mangandioxid und Selendioxid.

Die für beide Synthesen benötigten Ausgangsmaterialien sind nicht literaturbekannt. Die Verbindungen der Formel II erhält man jedoch leicht durch katalytische Hydrierung von Verbindungen der Formel

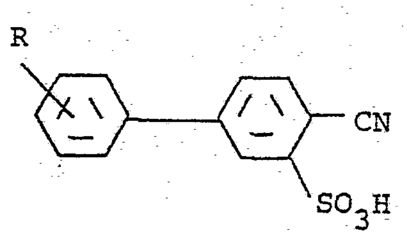

(IV)

in der R die angegebene Bedeutung hat, in Gegenwart von Raney-Nickel. Aus den Aminomethylverbindungen der Formel II lassen sich dann die entsprechenden Hydroxymethylverbindungen (III) durch Diazotieren und anschließendes "Verkochen" der

Diazoniumsalze gewinnen.

Zur Überführung der Aminomethylverbindung der Formel (II) in die Hydroxymethylverbindung der Formel (III) diazotiert man diese in einer Suspension in verdünnter Essigsäure bei 0 bis 20°C mit überschüssigem Natriumnitrit, zerstört den Nitrit-Überschuß und zersetzt die gebildete Diazoniumverbindung durch vorsichtiges Erwärmen der Mischung auf 60 bis 80°C.

Die erhaltenen Hydroxymethylverbindungen können durch Umetzung mit geeigneten Halogenierungsmitteln (z.B. $SOCl_2$) in die entsprechenden Chlormethylverbindungen überführt werden.

Schließlich läßt sich der Aldehyd der Formel (I), in der R Wasserstoff bedeutet, dadurch herstellen, daß man 2-Chlor-4-phenylbenzaldehyd der Formel

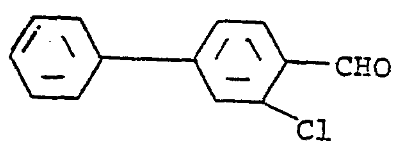

(V)

mit Natriumsulfit in Wasser bei 150 - 200°C umsetzt.

Die Reaktion wird im allgemeinen so durchgeführt, daß man eine Suspension des Aldehyds der Formel (V) in einer wäßrigen Lösung mindestens äquivalenter Mengen

Natriumsulfit mehrere Stunden in einem Autoklaven bei 150 - 200°C rührt.

Der Aldehyd der Formel (V) mit R=H ist bekannt (vgl. V.M. Berenfel'd, Zh. Obshch. Khim 32, 2169 - 77 (1962)).

Die Benzaldehyde der Formel I eignen sich beispielsweise zur Herstellung neuer wertvoller optischer Aufheller der Formel,

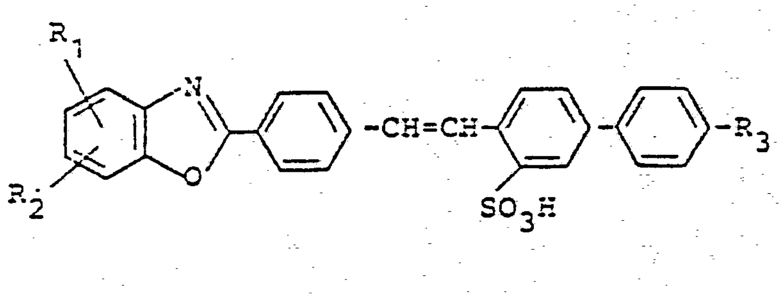 (VI)

worin,

$R_1$ Wasserstoff, $C_1$-$C_4$-Alkyl, $C_1$-$C_4$-Alkoxy, Chlor, COOH oder $SO_3H$,

$R_2$ Wasserstoff, $C_1$-$C_4$-Alkyl oder Chlor und

$R_3$ Wasserstoff, Chlor, COOH oder $SO_3H$ bedeuten.

Beispiel 1

26,3 g 4-Aminomethyl-biphenyl-3-sulfonsäure werden in 200 ml 80 %ige Essigsäure suspendiert. Hierauf werden 15 ml 30 %ige wäßrige Formalinlösung und dann 16,5 g Urotropin zugegeben. Es wird 4 Stunden unter Rückfluß gekocht, mit A-Kohle heiß klärfiltriert und das Lösungsmittel im Vakuum abdestilliert. Der schmierige Rückstand wird in wenig Wasser gelöst, die Lösung bei 60°C mit Kochsalz gesättigt, mit verdünnter Schwefelsäure angesäuert und auf ca 10°C abgekühlt. Hierbei fällt das Reaktionsprodukt der Formel

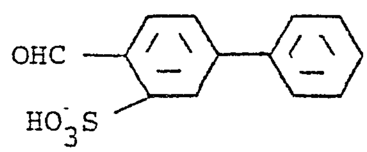

(1)

aus. Zur Überführung in das Natriumsalz wird der Filterrückstand mit Hilfe von Natronlauge in wenig Wasser gelöst und durch Zugabe von Kochsalz ausgesalzen. Nach Absaugen und Trocknen werden 17,5 g der Verbindung (1) als Natrium- Salz in Form eines weißen Pulvers erhalten. ($R_f$-Wert auf mit Kieselgel beschichteten Glasplatten : 0.52; Fließmittel: 30 ml Ammoniak, konz./ 50 ml Butylacetat/ 1o ml Wasser/ 70 ml Ethanol)

Die als Ausgangsprodukt benötigte 4-Aminomethyl-biphenyl-3-sulfonsäure wird wie folgt hergestellt:

100 g 4-Cyano-biphenyl-3-sulfonsäure, Na-Salz werden in 1500 ml 80 %igem Methanol heiß gelöst und dann in einem Autoklaven bei 60°C und 20 bar Wasserstoff-Druck

in Gegenwart von 20 g Raney-Nickel und 100 ml flüssigem Ammoniak hydriert. Nach Beendigung der Wasserstoffaufnahme wird auf Raumtemperatur abgekühlt, entspannt, vom Katalysator abfiltriert und das Lösungsmittel abdestilliert. Der feste Rückstand wird in wenig Wasser gelöst, klärfiltriert, durch Ansäuern ausgefällt, abgesaugt und getrocknet. Es werden 61 g 4-Aminomethyl-biphenyl-3-sulfonsäure der Formel

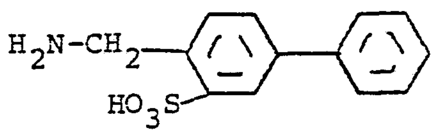

(2)

als weißes Pulver erhalten.

Beispiel 2

21,7 g 2-Chlor-4-phenylbenzaldehyd werden in einer Lösung von 28 g $Na_2SO_3 \cdot 7\ H_2O$ in 350 ml Wasser suspendiert und in einem Autoklaven 3 Stunden bei 190°C gerührt. Nach Abkühlen auf Raumtemperatur wird entspannt, die erhaltene Lösung in der Siedehitze klärfiltriert, angesäuert und mit 30 g Kochsalz versetzt. Beim Abkühlen auf Raumtemperatur fällt der Aldehyd der Formel (1) aus. Zur Überführung in das Na-Salz wird der Filterrückstand durch Zugabe von verdünnter Natronlauge in wenig Wasser bei 60°C gelöst. Man sättigt mit Kochsalz, kühlt auf Raumtemperatur ab und trocknet. Es werden 23,5 g der Verbindung der Formel (1) als Natrium-Salz in Form eines weißen Pulvers erhalten.

Beispiel 3

Biphenyl-4-aldehyd-4'-carbonsäure-3-sulfonsäure, Na-Salz

23 g (0,075 Mol) 4-Aminomethyl-biphenyl-4'-carbonsäure-3-sulfonsäure (VII), werden mit
35 g (0,25 Mol) Hexamethylentetramin [140] und
16 ml 37 %iger wäßriger Formalinlösung in
100 ml 60 %iger Essigsäure bei Rückflußtemperatur gerührt. Nach etwa 6-stündigem Rühren tritt allmählich Lösung ein; die Umsetzung wird dann im DC verfolgt. Wenn kaum noch Aminomethylverbindung zu erkennen ist (6 - 10 Stunden), destilliert man den größeren Teil der Essigsäure im Rotationsverdampfer unter vermindertem Druck ab, nimmt den Rückstand in Wasser auf, stellt auf etwa $p_H$ 3, klärt und salzt den gebildeten Aldehyd der Formel

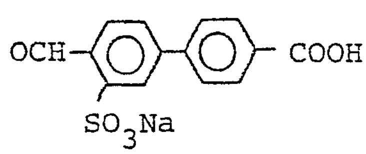

(3)

mit Kochsalz aus. (Bei weniger reinem Ausgangsmaterial oder bei zu langem Erhitzen neigt das Produkt zum Schmieren). Ausbeute 13 g; 56 % der Theorie. Weißes Kristallpulver.

Die als Ausgangsprodukt benötigte 4-Aminomethyl-biphenyl-4'-carbonsäure-3-sulfonsäure wird wie folgt hergestellt:

34 g (0,1 Mol) 4-Cyan-biphenyl-4'-carbonsäure-3-sulfonsäure, Na-Salz (VI) werden in 360 ml Wasser und 40 ml Ammoniak Konz. gelöst. Nach Zugabe von 12 g Raney-Nickel wird im VA-Rührautoklaven bei 35 - 40° mit 20 bar Wasserstoff hydriert. Nach Aufnahme der berechneten Menge kommt die Hydrierung zum Stillstand (ca. 6 Stunden); man saugt dann vom Katalysator ab (wieder einsetzbar), vertreibt die Hauptmenge des Ammoniaks durch Andestillieren im Vakuum und säuert die zurückbleibende helle Lösung mit verdünnter Salzsäure auf $p_H$ 4 - 5 an. Dabei fällt die Aminomethylsulfonsäure VII als inneres Salz in Form eines feinkristallinen Niederschlages aus, der abgesaugt, mit angesäuertem Wasser gewaschen und getrocknet wird (80°; Vakuum).

Es werden 26,5 g der Verbindung der Formel

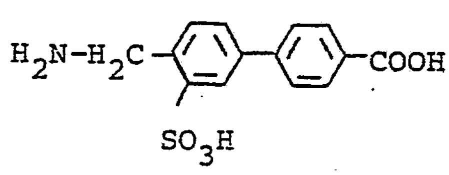

(4)

als weißes Kristallpulver erhalten.

Beispiel 4

6,9 g 2-(4'-Diethoxyphosphonmethylphenyl)-benzoxazol und 9,5 g 4-Phenylbenzaldehyd-2-sulfonsäure, Na-Salz (65 %ig) werden unter Durchleiten von Stickstoff in 100 ml Dimethylformamid auf 40°C erwärmt. In die entstandene Lösung werden bei 40 - 45°C innerhalb von 45 Minuten 11 g einer 30 %igen Natriummethylatlösung in Methanol getropft. Das Reaktionsgemisch wird noch 4 Stunden bei 45 - 50°C gerührt. Danach wird auf Raumtemperatur abgekühlt, mit 700 ml Wasser versetzt, zum Sieden erhitzt, abgesaugt und bei 100°C getrocknet. Man erhält 9,1 g (etwa 90 % der Theorie) der Verbindung der Formel

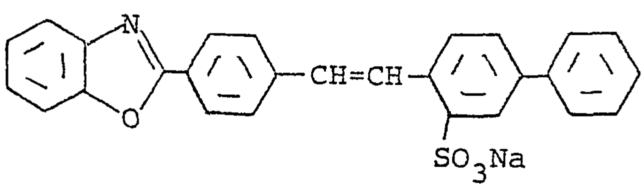

(5)

als gelbes Pulver.

Verwendet man anstelle von 2-(4'-Diethoxyphosphonomethylphenyl)-benzoxazol die aquivalente Menge 2-(4'-Diethoxyphosphonomethylphenyl)-5-methyl-benzoxazol der Formel

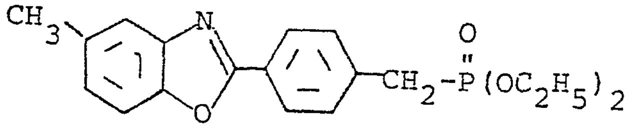

(6)

bzw. 2-(4'-Diethoxyphosphonomethylphenyl)-5,7-dimethyl-

benzoxazol der Formel

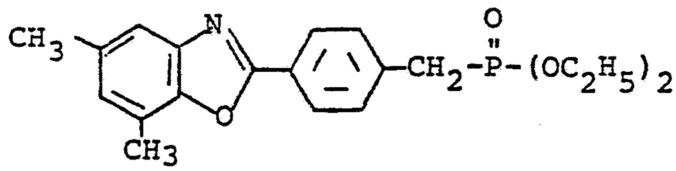

(7)

und verfährt im übrigen wie vorstehend beschrieben, so erhält man die Verbindung der Formel

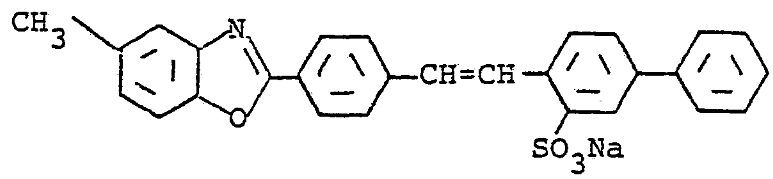

(8)

in Form eines gelben Pulvers bzw. die Verbindung der Formel

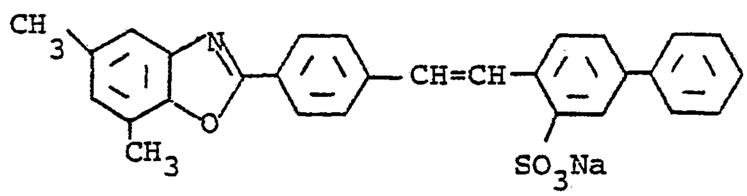

(9)

in Form eines gelben Pulvers.

Die Verbindungen lösen sich in Dimethylformamid mit neutral blauer Fluoreszenz am Tageslicht.

Patentansprüche

1) Verbindungen, die in Form der freien Säure der Formel

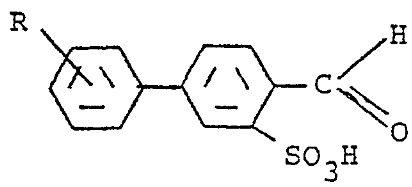

entsprechen,

worin R Wasserstoff, Halogen, eine gegebenenfalls abgewandelte Carbonsäure- oder Sulfonsäuregruppe ist.

2) Verbindungen gemäß Anspruch 1, dadurch gekennzeichnet, daß R Wasserstoff bedeutet.

3) Verfahren zur Herstellung der Verbindungen gemäß Anspruch 1, dadurch gekennzeichnet, daß man Verbindungen der Formel

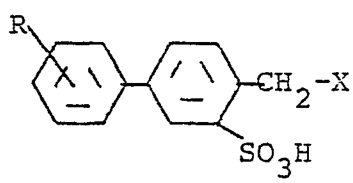

worin X für $NH_2$, OH oder Cl steht und R die obengenannte Bedeutung hat in an sich bekannter Weise oxidiert.